(19) Europäisches Patentamt
European Patent Office
Office européen des brevets

(11) Veröffentlichungsnummer: **0 626 448 A2**

# (12) EUROPÄISCHE PATENTANMELDUNG

(21) Anmeldenummer: **94107804.0**

(22) Anmeldetag: **19.05.94**

(51) Int. Cl.[5]: **C12N 15/21**, C12P 21/00, C12N 15/62

(30) Priorität: **26.05.93 DE 4317459**
**03.09.93 DE 4329756**

(43) Veröffentlichungstag der Anmeldung:
**30.11.94 Patentblatt 94/48**

(84) Benannte Vertragsstaaten:
**AT BE CH DE DK ES FR GB GR IE IT LI LU MC NL PT SE**

(71) Anmelder: **BOEHRINGER INGELHEIM INTERNATIONAL GmbH**
**Postfach 200**
**D-55216 Ingelheim (DE)**

(72) Erfinder: **Hauptmann, Rudolf, Dr.**
**Döllachgasse 22**
**A-2483 Ebrechsdorf (AT)**
Erfinder: **Falkner, Edgar, Dr.**
**Linienamtsgasse 4/2/14**
**A-1130 Wien (AT)**
Erfinder: **Bodo, Gerhard, Prof. Chem.**
**Hetzendorfer Strasse 116/1/4**
**A-1120 Wien (AT)**
Erfinder: **Voss, Tilman, Dr. Dipl.-Chem.**
**Weisses-Kreuz-Gasse 61**
**A-2340 Mödling (AT)**
Erfinder: **Maurer-Fogy, Ingrid, Dr.**
**Lindauergasse 35**
**A-1238 Wien (AT)**

## (54) Verfahren zur Herstellung und Reinigung von alpha-Interferon.

(57) Die Erfindung betrifft ein neues Herstellungsverfahren für rekombinantes IFN-α. Die Expression in *E. coli* erfolgt unter Kontrolle eines *pho*A-Promotors. Durch die Verknüpfung des IFN-α-Gens mit der STII-Signalsequenz wird die Sekretion des Proteins in den periplasmatischen Raum sowie ein korrekt prozessierter N-Terminus erreicht. Die Reinigung des Proteins erfolgt durch Adsorptionschromatographie auf Silicagel, hydrophobe Interaktionschromatographie, Kationen- sowie Anionenaustauschchromatographie.

EP 0 626 448 A2

Die Erfindung betrifft ein Herstellungsverfahren für Interferon-α (IFNα) durch bakterielle Expression und anschließende Isolierung, einen Expressionsvektor dafür sowie ein Verfahren zur Reinigung von IFNα.

Verfahren zur Herstellung von IFNα durch bakterielle Expression sind bekannt. Das übliche Verfahren beruht auf der cytoplasmatischen Expression des Proteins in *Escherichia coli*, bei dem das exprimierte IFNα entweder in unlöslicher Form in sogenannten Einschlußköpern in der Zelle vorliegt oder in der löslichen Fraktion nach dem Aufschließen der Zellwand gefunden wird (Thatcher *et* Panayotatos, 1986; Goeddel *et al.*, 1980; Dworkin-Rastl *et al.*, 1983). Die cytoplasmatische Expression weist allerdings Nachteile auf. Das synthetisierte Protein ist nicht korrekt gefaltet, und weil im Zytoplasma reduzierende Bedingungen herrschen, enthält es nicht die erforderlichen Disulfidbrücken. Das gebildete IFNα muß daher bei der Präparation oxidiert und umgefaltet werden. Dieser Prozeß ist ineffizient und führt zu unerwünschten Nebenprodukten (ganz- oder teilreduzierte Formen, Oligomere durch intermolekulare Disulfidbrückenbildung, fehlgefaltete Formen durch Ausbildung falscher Disulfidbrücken), die schwierig abzutrennen sind. Ein weiteres Problem ist, daß das N-terminale Methionin, mit dem die Translation beginnt, vom intrazellulär synthetisierten IFNα nur unvollständig abgespalten wird. Das daraus resultierende N-Met-IFNα kann vom nativen IFNα praktisch nicht abgetrennt werden.

Ein weiterer Nachteil gegenwärtig benutzter Verfahren ist die Verwendung von Promotoren, die in nicht-induziertem Zustand nicht vollständig abgeschaltet sind, die durch Zugabe von Chemikalien induziert werden müssen, und deren Expressionsrate im induzierten Zustand nicht befriedigend ist, wie z.B. der *trp*-Promotor aus *Serratia marcescens*.

Um einige der genannten Nachteile zu überwinden und trotzdem das ökonomische *E.−coli*-System zu nutzen, versuchten Breitling *et al.* (Breitling *et al.*, 1989) IFNα1 und ein IFNα 1/2-Hybrid mit einem Vektor zu exprimieren, der die Sekretion des Interferons durch die Zellmembran in den periplasmatischen Raum ermöglichte. Sie verwendeten dabei Promotor, Ribosomenbindungsstelle (RBS) und Signalsequenz eines bakteriellen Staphylokinasegens (*sak*42D). 60-80% des so hergestellten IFNα wurden in den periplasmatischen Raum sezerniert. Das Protein enthielt allerdings, bedingt durch das Vektorkonstrukt, zusätzliche N-terminale Aminosäuren, die im entsprechenden nativen IFNα nicht vorkommen. Als gravierender Nachteil dieses Expressionssystems erwies sich indes die Tatsache, daß die mit diesem Konstrukt transformierten Stämme genetisch nicht stabil blieben; die Expressionskassette wurde durch die spontane Insertion eines IS1-Elements desaktiviert. Die Aufgabe, ein Expressions/Sekretionssystem in *E. coli* für die Herstellung von humanem IFNα bereitzustellen, war im Stand der Technik also ungelöst.

Als eine Expressions/Sekretions-Kassette, die im Falle der Expression des menschlichen Wachstumsfaktor-Rezeptors in *E. coli* zum Erfolg geführt hatte, war ein Konstrukt aus dem Promotor der alkalischen Phosphatase (*pho*A) und der Signalsequenz des hitzestabilen Enterotoxins II (STII) bekannt (Fuh *et al.*, 1990).

Ein weiteres Problem bei der Herstellung von rekombinantem IFNα in *E. coli* ist die Reinigung des Proteins aus dem Bakterienlysat. Hier sind eine Reihe von Verfahren bekannt (Thatcher *et* Panayotatos, 1986; EP-A 203 382). Um die native Faltung des Proteins zu erhalten, sind dabei Verfahren vorzuziehen, die ohne Denaturierungs- und Fällungsschritte auskommen. Ein solches Verfahren wird in der EP-A 396 555 beschrieben. Es besteht aus den Schritten Immunoaffinitätschromatographie, Reversed-Phase-Chromatographie (RPC), Kationenaustauschchromatographie, Konzentrierung durch Ultrafiltration und Gelfiltrationschromatographie. Dieses Verfahren beruht wie andere bekannte Verfahren auf der hohen Selektivität der Immunoaffinitätschromatographie im ersten Schritt. Es ist kein Verfahren zur Herstellung von hochgereinigtem IFNα, insbesondere IFNα2, bekannt, das ohne Denaturierungs-/Fällungsschritte und ohne Immunoaffinitätschromatographie auskommt. Gleichzeitig ist ein solches Verfahren aus ökonomischen und technischen Gründen wünschenswert. Wegen der für die Immunoaffinitätschromatographie notwendigen monoklonalen Antikörper sind ihre Kosten hoch, gleichzeitig ist, da die Lebensdauer der antikörpergekoppelten Matrices endlich ist, eine kontinuierliche Versorgung mit diesen Antikörpern notwendig.

Aufgabe der Erfindung ist die Bereitstellung eines wirtschaftlicheren und leistungsfähigeren Verfahrens zur Herstellung von Interferon-α, insbesondere Interferon-α2, durch rekombinante Expression in *E. coli.* Dabei mußte das Problem gelöst werden, ein effizientes und stabiles System zur Expression/Sekretion des Proteins in den periplasmatischen Raum oder das Kulturmedium zu etablieren. Ferner war ein Verfahren zu entwickeln, das exprimiertes Protein schonend ohne Denaturierungs-/Fällungsschritte und ohne die Notwendigkeit der Immunoaffinitätschromatographie hochreinigen kann.

Diese Aufgabe konnte mit der vorliegenden Erfindung gelöst werden. Die Etablierung eines stabilen Expressions/Sekretionssystems für IFNα in *E. coli* gelang durch die Konstruktion eines Vektors, der die Signalsequenz (Leadersequenz) des hitzestabilen Enterotoxins II (STII) aus *E. coli* verknüpft mit der kodierenden Sequenz für ein reifes menschliches Interferon-α, vorzugsweise Interferon-α2, enthält. Bevorzugt erfolgt die Expressionskontrolle mittels des Promotors der alkalischen Phosphatase aus *E. coli* (*pho*A).

Als vorteilhaft erwies sich ferner die Integration der Ribosomenbindungsstelle des STII-Gens. Ein weiterer überraschender Fortschritt konnte durch die Bereitstellung eines Reinigungsverfahrens für Interferon-α, das aus den Schritten Adsorptionschromatographie auf Silicagel, hydrophobe Interaktionschromatographie (HIC), Kationenaustauschchromatographie und Anionenaustauschchromatographie besteht, erreicht werden.

Ein Aspekt der Erfindung betrifft somit ein Verfahren der Herstellung von IFNα durch bakterielle Expression, bei dem transformierte Bakterienzellen verwendet werden, die einen Expressionsvektor enthalten, in dem die STII-Signalsequenz mit einem IFNα-Gen verknüpft ist, und durch Isolierung des exprimierten IFNα. Ein weiterer Aspekt betrifft einen bakteriellen Expressionsvektor für die Herstellung von IFNα, der ein Konstrukt aus der Signalsequenz des STII-Gens und einem IFNα-Gen enthält, sowie die Verwendung eines solchen Vektors zur Herstellung von IFNα. Ein dritter Aspekt betrifft ein Verfahren zur Reinigung von IFNα durch die chromatographischen Schritte Adsorptionschromatographie auf Silicagel, hydrophobe Interaktionschromatographie, Kationen- sowie Anionenaustauschchromatographie.

Als Ausgangspunkt für die Konstruktion des Vektors kann ein in *E. coli* replikationsfähiges Plasmid dienen, beispielsweise eignet sich das Plasmid pAT153 (Twigg *et al.*, 1980) sehr gut für diesen Zweck. Eine Nukleotidsequenz, die für das Signalpeptid des STII-Gens kodiert, ist Stand der Technik (Picken *et al.*, 1983; Lee *et al.*, 1983). Der Fachmann ist in der Lage, durch Mutationen (Substitution, Deletion, Insertion, Addition) Varianten dieser Sequenz herzustellen, ohne ihre Grundeigenschaften zu verändern, und insbesondere solche Nukleotidsequenzen herzustellen, die wegen der Degeneration des genetischen Codes für die gleiche Aminosäuresequenz des Signalpeptids kodieren (Sambrook *et al.*, 1989, bes. Kapitel 15). Eine ganze Reihe von Sequenzen, die für Mitglieder der IFNα-Familie kodieren, ist bekannt (Mantei *et al.*, 1980; Streuli *et al.*, 1980; Goeddel *et al.*, 1981); die Homologie der sie kodierenden Gene beträgt mehr als 70 %. Weitere Varianten dieser Sequenzen können in der Natur gefunden werden oder mit Methoden aus dem Stand der Technik, z.B. durch Mutagenese, aus den bekannten Sequenzen hergestellt werden (Sambrook *et al.*, 1989, bes. Kapitel 15). Der Begriff "IFNα" im Sinne der Erfindung schließt demzufolge neben den bekannten Sequenzen auch solche Varianten ein, deren Gene durch hohe Homologie zu den bekannten Sequenzen gekennzeichnet sind und die für biologisch aktives IFNα kodieren. Besonders bevorzugt ist dabei die Sequenz, die für IFNα2c kodiert (Dworkin-Rastl *et al.*, 1983; Bodo *et* Fogy, 1985). Besonders bevorzugt ist ferner die Verwendung des *pho*A-Promotors zur Kontrolle der Expression und darüber hinaus vorteilhaft die Integration der Ribosomenbindungsstelle des STII-Gens. Die Sequenz des *pho*A-Promotors (Chang *et al.*, 1986; Shuttleworth *et al.*, 1981) sowie der STII-Ribosomenbindungsstelle (Picken *et al.*, 1983; Lee *et al.*, 1983) sind bekannt; auch aus diesen Sequenzen kann der Fachmann ohne weiteres äquivalente Varianten herstellen. Konstruktion des Vektors, Transformation geeigneter *E. coli*-Stämme, Fermentation sowie Extraktion können nach an sich bekannten Methoden erfolgen (Sambrook *et al.*, 1989). Für die Expression ist beispielsweise der *E. coli*-Stamm W3110 (*E. coli* K12 Wildtyp $f^-$, $\lambda^-$, IN (rrnD-rrnE)1) gut geeignet. Die Vorkultur kann gut in LB-Medium, die Hauptkultur unter Kontrolle von Sauerstoff- und Nährstoffzufuhr bis zu einer $OD_{546}$ von 250 bis 280 erfolgen. Als gut geeignet erwies sich ein Extraktionsverfahren, bei dem säureinaktivierte Biomasse in verdünnter Essigsäure mit Hilfe eines Homogenisators suspendiert, mit Polyethylenimin, bevorzugt in einer Konzentration von 0.25% (w/v) versetzt, auf alkalischen pH, vorzugsweise pH 10, eingestellt, gerührt und anschließend durch Zentrifugation die Bakterien abgetrennt wurden. Die Reinigung kann nach an sich bekannten Verfahren erfolgen (Thatcher *et* Panayotatos, 1986; EP-A 203 382). Besonders vorteilhaft ist jedoch ein Reinigungsverfahren mit vier chromatographischen Schritten, und zwar Adsorptionschromatographie auf Silicagel, hydrophobe Interaktionschromatographie, Kationen- und Anionenaustauschchromatographie. Als Gelbett der Silicachromatographie erwies sich das vom Typ 953W der Firma Grace gut geeignet, als Elutionsmittel war ein Puffer, der 500 - 1500 mM Tetramethylammoniumchlorid (TMAC), bevorzugt 800 mM TMAC, vorteilhaft verwendbar. Für die hydrophobe Interaktionschromatographie erwies sich eine Phenylsepharose als gut zu verwendendes Gelbett. Der Probenauftrag erfolgte vorzugsweise in Anwesenheit von 20% Ammoniumsulfat, die Säule war mit einem Puffer, der 30% Ammoniumsulfat enthielt, equilibriert worden. Das IFNα wurde mit einem linearen Gradienten mit einer Endkonzentration von 30% Ethylenglykol eluiert. Die Kationenaustauschchromatographie konnte sehr gut mit einem Sulfopropyl-Ionenaustauscherharz ausgeführt werden. Der Probenauftrag erfolgte bei einem pH-Wert von 3-5, vorzugsweise pH 3, die Säule war auf pH 5 equilibriert. IFNα konnte erfolgreich mit einem linearen Kochsalzgradienten mit einem Zusatz von 10% Ethylenglykol eluiert werden. Als Gelbett für die Anionenaustauschchromatographie war DEAE-Sepharose sehr vorteilhaft zu verwenden, Auftrag und Elution erfolgten bei pH 5.5 - 6.0, vorzugsweise bei pH 5.8. Zur Elution war ein linearer Kochsalzgradient mit einem Zusatz von 0.1% Tween 20 gut geeignet. Es gehört zu den technischen Möglichkeiten des Fachmanns, ohne erfinderische Tätigkeit jeweils eines oder mehrere Gelmaterialien durch gleichwertige zu ersetzen, die auf den gleichen Trennprinzipien basieren, und auf diese Weise das erfindungsgemäße Verfahren äquivalent auszuführen.

Überraschenderweise konnte mit der Verknüpfung der STII-Signalsequenz mit dem IFNα-Gen ein stabiles Expressions/Sekretionssystem etabliert werden, was mit der vorbeschriebenen *sak*42D-Leader /IFNα-Kombination nicht gelungen war. Als besonders erfolgreich erwies sich die Expression dieser Sequenz unter der Kontrolle des *pho*A-Promotors. Die Integration der Ribosomenbindungsstelle des STII-Gens erwies sich in diesem Zusammenhang als zusätzlich vorteilhaft. Die Expression kann über die Kontrolle der Phosphatkonzentration im Medium (Phosphatmangel aktiviert den *pho*A-Promotor) zuverlässig gesteuert werden; im inaktivierten Zustand gibt es keine nachweisbare Basalexpression. Zusätzliche Chemikalien brauchen zur Aktivierung nicht zugegeben zu werden, die Expressionsrate im aktivierten Zustand ist hoch. Das synthetisierte Protein wird in hohen Anteilen in den periplasmatischen Raum sezerniert. Das sezernierte Protein ist korrekt gefaltet, enthält den authentischen N-Terminus und die richtigen Disulfidbrücken. Die SDS-Gelanalyse der Expression in *E. coli* W3110 zeigte, daß 30-50% des synthetisierten IFNα korrekt prozessiert waren, dies entspricht praktisch dem kompletten Anteil des sezernierten Proteins.

Mit dem in Beispiel 3 beschriebenen Extraktionsverfahren konnten 29.3 ± 5.9% des insgesamt in der Biomasse nachweisbaren IFNα2c extrahiert werden. Dies entsprach dem beobachteten Prozessierungsgrad von 30-50%. Der Extrakt aus der Biomasse enthielt 4.5 ± 1.8% IFNα2c, bezogen auf Gesamtprotein. Die Silica-Adsorptionschromatographie führte zu einem IFNα2c-Pool mit einer durchschnittlichen Reinheit von 16.7 ± 4.4%. Die Phenyl-Sepharose-Chromatographie mit einer Ausbeute von 93.2 ± 7.3% ergab ein IFNα2c mit einer Reinheit von 71.2 ± 15.5%. Die Sulfopropyl-Ionenaustauschchromatographie erbrachte eine Ausbeute von 70.9 ± 14.8% und eine Reinheit von 97.6 ± 4.6%. Der letzte Schritt, die DEAE-Ionenaustauschchromatographie, führte bei einer Ausbeute von 86.9 ± 9.2% zu 100% reinem IFNα2c, wie unten charakterisiert. Die Daten aus 6 verschiedenen Reinigungen sind in den Tabellen 1 (Ausbeuten) und 2 (IFNα2c-Gehalt) zusammengefaßt. Fig. 3 zeigt charakteristische Chromatogramme von jedem Reinigungsschritt.

Aus 1 kg Biomasse wurden 340 ± 100 mg gereinigtes IFNα2c erhalten. Die Ausbeute des Reinigungsprozesses ist 56.1 ± 22.2%. Die Gesamtausbeute, bezogen auf den IFNα2c-Gehalt der Biomasse ist 14.4%. Diese Daten sind in Tabelle 3 zusammengefaßt. Fig. 4 zeigt eine typische SDS-PAGE von gereinigtem IFNα2c, eluiert beim letzten chromatographischen Schritt. Die 18-kDa-Bande von IFNα2c ist die einzige sichtbare Bande. Kontaminierende Banden werden nicht beobachtet. Fig. 5A zeigt ein typisches Reversed-Phase-HPLC-Chromatogramm. Das gereinigte IFNα2c eluiert als homogener Peak bei 24.8 Minuten. Wurde dieses Material mit einem flachen Acetonitrilgradienten eluiert (Fig. 5B), wurden 2 Kontaminationspeaks an beiden Seiten des Hauptpeaks beobachtet. Diese Schultern, die etwa 1.8% des Gesamt-IFNα2c-Gehaltes enthalten, repräsentieren Formen, die am Methionin 111 oxidiert (erste Schulter) oder am N-Terminus acetyliert (zweite Schulter) sind.

***Tabelle 1**: Ausbeuten verschiedener Reinigungsschritte in Prozent IFNα2, die nach dem jeweiligen Reinigungsschritt erhalten wurden, dargestellt für 6 verschiedene Reinigungsprozeduren (p1-p6) aus 6 verschiedenen Biomassen. Die letzten beiden Spalten enthalten den Mittelwert (M) und die Standardabweichung (sd)*

| | p1 | p2 | p3 | p4 | p5 | p6 | M | sd |
|---|---|---|---|---|---|---|---|---|
| Extrakt | 37.9 | 24.0 | 34.3 | 30.7 | 29.1 | 20.0 | 29.3 | 5.9 |
| Silica | 62.0 | 95.8 | 88.2 | 99.5 | 74.1 | 81.0 | 83.4 | 12.8 |
| Phenyl | 100.0 | 82.2 | 85.9 | 100.0 | 100.0 | 91.0 | 93.2 | 7.3 |
| Sulfopro | 64.0 | 54.3 | 76.5 | 100.0 | 60.0 | 71.0 | 70.9 | 14.8 |
| DEAE | 95.0 | 100.0 | 83.5 | 88.2 | 84.0 | 71.0 | 86.9 | 9.2 |

***Tabelle 2**: IFNα2-Gehalt verschiedener Reinigungsschritte. Die Daten sind als Prozentsatz des IFNα2-Gehalts, bezogen auf den Gesamtproteingehalt, der bei diesem Reinigungsschritt erhalten wurde, so dargestellt wie in Tabelle 1.*

| | p1 | p2 | p3 | p4 | p5 | p6 | M | sd |
|---|---|---|---|---|---|---|---|---|
| Extrakt | 8.0 | 2.1 | 4.1 | 4.7 | 3.6 | 4.4 | 4.5 | 1.8 |
| Silica | 12.9 | 11.6 | 15.7 | 15.7 | 19.4 | 15.6 | 16.7 | 4.4 |
| Phenyl | 76.6 | 43.3 | 62.9 | 62.9 | 80.0 | 93.5 | 71.2 | 15.5 |
| Sulfopro | 98.5 | 87.3 | 100.0 | 100.0 | 100.0 | 100.0 | 97.6 | 4.6 |
| DEAE | 100.0 | 100.0 | 100.0 | 100.0 | 100.0 | 100.0 | 100.0 | 0.0 |

***Tabelle 3:** Gesamtausbeuten des Reinigungsverfahrens. Der IFNα2c-Gehalt der **Biomasse** ist als g IFNα2/kg Biomasse dargestellt. **Prozessierung** und **Extraktion** sind als Prozentsatz des Gesamtgehalts an IFNα2 ausgedrückt. Die Ausbeute der **Reinigung** ist dargestellt als Prozentsatz von IFNα2c relativ zum IFNα2-Gehalt des Extrakts. Die Gesamtausbeute ist in mg IFNα2, erhalten pro kg Biomasse, und als Prozentsatz von gereinigtem IFNα2c, bezogen auf den IFNα2c-Gehalt des Extrakts, ausgedrückt.*

| | p1 | p2 | p3 | p4 | p5 | p6 | M | sd |
|---|---|---|---|---|---|---|---|---|
| Biomasse [g/kg] | 1.4 | 1.0 | 1.1 | 1.5 | 1.1 | 1.8 | 1.3 | 0.2 |
| Prozessierung [%] | 50 | 40 | 40 | 40 | 20 | 40 | 38.3 | 8.9 |
| Extraktion [%] | 37.9 | 24.0 | 34.3 | 30.7 | 29.1 | 20.0 | 29.3 | 4.7 |
| Reinigung [%] | 39.7 | 42.7 | 57.9 | 90 | 44.5 | 52.3 | 56.1 | 22.2 |
| Gesamtausbeute [mg] | 538 | 206 | 366 | 480 | 280 | 258 | 340 | 120 |
| Gesamtausbeute [%] | 14.3 | 20.3 | 16.6 | 23.9 | 10.9 | 7.4 | 14.4 | 6.9 |

**Abbildungen**

***Fig. 1:***

*A)* Genkarte von pCF2. Das *EcoRI–Bam*HI-Fragment von pAT153 wurde durch die Expressionskassette für IFNω 1 ersetzt.

*B)* Sequenz des *Eco*RI(zerstört)-*Bam*HI-Teils, der den *pho*A-Promotor, STII-Leader + IFNω 1-Gen enthält.

***Fig. 2:***

*A)* Genkarte des Plasmids pDH13. Das *Ssp*I-*Pst*I-Fragment von pAT153 wurde durch die IFNα2c-Expressionskassette (*Eco*RI-*Pst*I-Fragment von 2*B*)) ersetzt. Das ß-Lactamase-Gen ist zerstört.

*B)* Nukleotidsequenz des *Eco*RI-*Hind*III-Inserts von pDH13.

***Fig. 3:*** Chromatographische Reinigung von IFNα2c, extrahiert aus Biomasse.

*A)* Adsorptionschromatographie auf Silicagel. Der Pfeil zeigt die Elution mit 800 mM Tetramethylammoniumchlorid an.

*B)* Hydrophobe Interaktionschromatographie auf Phenylsepharose. Die Elution wurde mit einem linearen Gradienten von 0 bis 100% Lösungsmittel B wie angezeigt ( ----) durchgeführt.

*C)* Sulfopropyl-Kationenaustauschchromatographie. Die Elution wurde mit einem Gradienten von 0 bis 100% Lösungsmittel B wie angezeigt (----) ausgeführt.

*D)* Anionenaustauschchromatographie auf DEAE-Sepharose. Die Elution wurde mit einem Gradienten von 0 bis 100% Lösungsmittel B wie angezeigt (----) durchgeführt.

Die Balken unter den Hauptpeaks in jedem Chromatogramm zeigen die IFNα2-haltigen Pools an, die gesammelt und für die folgenden Schritte verwendet wurden.

***Fig. 4:*** SDS-PAGE von gereinigtem IFNα2c, gefärbt mit Coomassie Blue. Die Zahlen am linken Rand zeigen die Molekulargewichte der Standardproteine an.

*Spur 1:* IFNα2c-Standard
*Spur 2:*3 µg IFNα2c
*Spur 3:*6 µg IFNα2c
*Spur M:* Molekulargewichtsstandard

***Fig. 5:*** Charakterisierung von gereingtem IFNα2c durch Reversed Phase HPLC.

*A)* Elution von IFNα2c mit einem linearen Gradienten von 20-68% Lösungsmittel B in 24 Minuten.

*B)* Elution von IFNα2c mit einem linearen Gradienten von 45-53% Lösungsmittel B in 30 Minuten.

## Beispiele

### Beispiel 1: Herstellung des Expressionsvektors pDH13 sowie damit transformierter Zellen

*Allgemeine Methoden*

Restriktionsverdau von DNA mit Restriktionsendonukleasen, Auffüllreaktionen, Phenolextraktion und Fällung von DNA, Agarose-Gelelektrophorese und Elution von DNA aus Agarosegelen, Ligation von DNA-Molekülen, Transformation von Bakterien und Plasmidisolierung aus Bakterien sind Standardverfahren und wurden durchgeführt wie von Sambrook *et al.* (1989) beschrieben.

*Plasmide*

pCF2 pCF2 wurde aus dem Plasmid pAT153 (Twigg *et al.*, 1980) hergestellt. Es enthält den Promotor der alkalischen Phosphatase aus *E. coli* (*pho*A, Chang *et al.*, 1986; Schuttleworth *et al.*, 1986), die kodierende Region des STII-Leaderpeptids (Picken *et al.*, 1983; Lee *et al.*, 1983) sowie das Gen für menschliches IFNω1 (Hauptmann *et al.*, 1985). Fig. 1 zeigt die Genkarte von pCF2 sowie die Sequenz des relevanten Abschnitts.

pER21/1 pER 21/1 ist ein bakterieller Expressionsvektor für IFNα2c, dessen Herstellung in der EP 0 115 613 beschrieben wird.

*Oligonukleotide (5′ →3′):*

EBI-2787: CGTCTTCAAGAATTCGAGATTATCG
EBI-2799: GGCAGATCACATGCATAGGCATTTGTAGCAATAG
EBI-2798: ATGCCTATGCATGTGATCTGCCTCAAACCCACAGC
EBI-2797:

GACTTCAGAAGCTTCTGCAGTTACGATCGTTATCATTCCTTAC
TTCTTAAACTTTC

*Herstellung der Expressionskassette aus phoA–Promotor, IFNα2c–Sequenz und STII–Leadersequenz in einer Zweischritt–PCR*

pER21/1-DNA wurde mit *Hind*III linearisiert, pCF2-DNA mit *Pvu*I. Die im folgenden verwendete Methode ist als SOE-PCR beschrieben ("splicing by overlap extension", Ho *et al.*, 1989).

***PCR 1a** (Amplifikation des IFNα2c–Gens):* 100 ng linearisierter pER21/1-DNA, 25 pmol EBI-2797 und 25 pmol EBI-2798 wurden in 50 µl Puffer, der 50 mM KCl, 10 mM Tris-HCl, pH 8.3, 1.5 mM $MgCl_2$, 0.01% Gelatine, 0.2 mM dATP, 0.2 mM dGTP, 0.2 mM dCTP, 0.2 mM dTTP und 1.25 Einheiten *Taq*-Polymerase enthielt, in einem Perkin Elmer Cetus Thermocycler TC-1 Thermozyklen unterworfen. Nach 3 min Inkubation bei 94 °C wurden 10 Stufenzyklen (Stufe 1: 40 sec bei 94 °C, Stufe 2: 30 sec bei 55 °C, Stufe 3: 90 sec bei 72 °C) ausgeführt.

***PCR 1b** (Amplifikation von phoA–Promotor plus STII–Leadersequenz):* 100 ng linearisierter pCF2-DNA, 25 pmol EBI-2787 und 25 pmol EBI 2799 wurden im gleichen Puffer und unter gleichen Bedingungen wie unter PCR 1a beschrieben Thermozyklen unterworfen.

Die resultierenden DNA-Fragmente von PCR 1a (540bp) und PCR 1b (374 bp) wurden gelgereinigt (1.2% low gelling type Agarose in TBE-Puffer, 1 x TBE: 10.8 g Tris/l, 5.5 g Borsäure/l, 0.93 g EDTA/l). Das Agarosestückchen, das das jeweilige DNA-Fragment enthielt, wurde ausgeschnitten und die Agarose geschmolzen, indem 100 µl $H_2O$ zugegeben und auf 70 °C erhitzt wurde.

***PCR 2:*** 5 µl von jeder Agarose/DNA-Lösung wurden vereinigt und in 100 µl Lösung, die jeweils 50 pmol von EBI-2787 und EBI-2797 enthielt, Thermozyklen unterworfen. Der Puffer war der gleiche wie unter PCR 1a beschrieben. Das Thermozyklusgerät wurde so programmiert, daß an eine Verzögerungszeit von 5 min bei 94 °C 20 Stufenzyklen (Stufe 1: 40 sec bei 94 °C, Stufe 2: 30 sec bei 55 °C, Stufe 3: 5 min bei 72 °C; Stufe 3 wurde bei jedem neuen Zyklus um 5 Sekunden verlängert) angeschlossen wurden. Nach der Amplifikation wurde die DNA durch Phenol/Chloroform-Extraktion und Ethanol-Präzipitation gereinigt. Das PCR-Produkt wurde aufgelöst und mit *Hind*/III und *Eco*RI in den entsprechenden Puffern geschnitten.

*Klonierung des PCR–Produktes (pDH9)*

Bluescribe M13$^+$ (Stratagene, San Diego, CA, USA) wurde mit *Hind*III und *Eco*RI doppelt geschnitten und das große Fragment wurde mit einem 1.2%igen Agarosegel gelgereinigt. 10 ng Bluescribe M13$^+$ DNA und 50 ng mit *Eco*RI/*Hind* III geschnittenes PCR-Produkt wurden in 10 µl Lösung, die 50 mM Tris-HCl, pH 7.6, 10 mM $MgCl_2$, 20 mM Dithiothreitol, 1 mM ATP, 50 µg/ml Rinderserumalbumin (BSA) und 2 Einheiten T4-DNA-Ligase (NEN) enthielt, 1 Stunde bei 0 °C und 3 Stunden bei Raumtemperatur ligiert. 8 µl dieser Lösung wurden für die Transformation kompetenter *E. coli*-Zellen vom Stamm JM 101 (*E. coli* K12, *Sup*E, *thi*, Δ(*lac*–*pro*AB), [F', *tra*D36, *pro*AB, *lac*IZΔM15]) verwendet.

Ein Klon wurde ausgewählt, die DNA isoliert und die Expressionskassette sequenziert. Die Sequenz entsprach genau der theoretisch erwarteten Sequenz (Fig. 2). Das Plasmid wurde als pDH9 bezeichnet.

*Konstruktion des Expressionsplasmids **pDH13***

pAT153 wurde mit *Ssp*I und *Pst*I doppelt geschnitten und das große Fragment wurde isoliert. pDH9 wurde mit *Eco* RI geschnitten und die Enden aufgefüllt unter Verwendung des Klenowfragments der DNA-Polymerase I und der 4 dNTPs. Nach Phenolextraktion und Fällung der linearen pDH9-DNA wurde diese DNA mit *Pst* I geschnitten und das Fragment, das den *pho*A-Promotor, die STII- Leadersequenz und das IFNα2c-Gen enthielt, aus einem 1%igen Agarosegel isoliert.

10 ng pAT153 x *Ssp*I x *Pst*I und 30 ng des Fragments, das die Expressionskassette enthielt, wurden in 10 µl Lösung für 5 Stunden bei Raumtemperatur ligiert. 5 µl von diesem Ansatz wurden verwendet, um kompetente *E. coli*-Bakterien des Stammes HB101 zu transformieren. Die Selektion der transformierten Bakterien wurde auf LB-Agarplatten (10 g Trypton/l, 5 g Hefeextrakt/l, 5 g NaCl/l, 15 g Bacto-Agar/l) durchgeführt, die 10 µg/ml Tetracyclin enthielten. Eine Genkarte von pDH13 und die Sequenz der relevanten Region ist in Fig. 2 dargestellt.

Plasmid-DNA verschiedener so erhaltener Kolonien wurde isoliert und durch Restriktionsanalyse auf korrekte Zusammensetzung überprüft. Ein Plasmid wurde ausgewählt und als pDH13 bezeichnet. Das Plasmid pDH13 wurde zur Transformation von *E. coli* W3110 (*E. coli* K12 Wildtyp, f$^-$, λ$^-$, IN (*rrn*D-*rrn*E)1) verwendet.

**Beispiel 2: Fermentation**

*Vorkultur*

700 ml autoklaviertes LB-Medium (10 g Bacto-Trypton/l, 5 g Bacto-Hefeextrakt/l, 10 g NaCl/l, pH 7.0), das 5 mg/l Tetracyclin enthielt, wurden in einem 2l-Glasgefäß aus einer Stockkultur so beimpft, daß eine $OD_{546}$ von 0.01 erhalten wurde. Die Kultur wurde 10 Stunden bei 37 °C unter starkem Rühren (800 U/min) und Belüftung (5 Fermentervolumnia pro Minute [vvm]) inkubiert.

*Hauptkultur*

Mediumzusammensetzung

im Fermenter:

1.21 g/l $(NH_4)_2HPO_4$
3.96 g/l $(NH_4)_2SO_4$
6.53 g/l $K_2HPO_4$
1.23 g/l $MgSO_4$ x 7 $H_2O$
0.32 g/l NaCl
0.25 g/l $NH_4Cl$
1.0 g/l $Na_3$-Citrat x 2 $H_2O$
1.0 ml/l Spurenelementekonzentrat
12.5 g/l Glucose
20 mg/l Thiamin-HCl
50 mg/l L-Tryptophan
100 mg/l L-Leucin
50 mg/l L-Methionin
5 mg/l Tetracyclin

Spurenelementekonzentrat:

(Mengenangaben pro 100 ml)

3.35 g $FeCl_3$ x 6 $H_2O$
1.09 g $ZnSO_4$ *x* 7 $H_2O$
0.267 g $CoCl_2$ x 6 $H_2O$
0.267 g $Na_2MoO_4$ x 2 $H_2O$
0.221 g $CuSO_4$ x 5 $H_2O$
0.333 g $H_3BO_3$
1.37 g $MnSO_4$ x $H_2O$
10 ml HCl conc.
$H_2O$ *ad* 100 ml

Fütterung während der Fermentation:

(Mengen bezogen auf Fermentervolumen)

350 g/l Glucose
3.70 g/l $MgSO_4$ x 7 $H_2O$
175 mg/l Thiamin-HCl
0.50 g/l L-Tryptophan
4.0 g/l L-Leucin
2.0 g/l L-Methionin

Zudosierung von Antischaummittel während der Fermentation:

(bezogen auf Fermentervolumen)

1.0 ml/l UCON LB625

Salze ($(NH_4)_2PO_4$, $(NH_4)_2SO_4$, $K_2HPO_4$, NaCl, $NH_4Cl$ und Na-Citrat) wurden in einem Fermenter sterilisiert. Spurenelemente, $MgSO_4$, Glucose, Thiamin, L-Tretophan, L-Leucin, L-Methionin und Tetracyclin wurden nach Abkühlung aseptisch so zugegeben, daß ein Startvolumen von 7 Litern erhalten wurde. 600 ml der Vorkultur wurden automatisch in den Fermenter überimpft. Die Fermentationsbedingungen waren: Rühren bei 1000 U/min, Belüftung von 1 vvm, 0.3 bar Überdruck, eine Temperatur von 37.0 ± 0.1 °C, der pH wurde auf 6.7 ± 0.1 mit $NH_3$ und $H_2SO_4$ gehalten. Die Konzentration an gelöstem Sauerstoff wurde durch Belüftung mit sauerstoffangereicherter Luft nach Bedarf oberhalb von 15% Luftsättigung (bei 0.3 bar Überdruck) gehalten. Nach Verbrauch der anfänglich vorhandenen Glucose wurde eine Fütterungsprozedur gestartet, die durch die Sauerstoffkonzentration automatisch ausgelöst wurde und Glucose, Thiamin, $MgSO_4$, L-Tryptophan, L-Leucin und L-Methionin enthielt. Die Fütterungsgeschwindigkeit begann mit 2.5 g/l/h Glucose und wurde innerhalb von 24 Stunden kontinuierlich auf 5.0 g/l/h gesteigert und anschließend bis zum Ende des Fermentationsprozesses konstant gehalten.

Die Fermentation wurde beendet, nachdem eine Gesamtmenge von 350 g/l Glucose zugegeben worden war. Zu diesem Zeitpunkt war eine typische optische Dichte von 250 bis 280 bei 546 nm erreicht.

Zur Inaktivierung der Biomasse wurde der Ansatz auf etwa 10 °C gekühlt und gleichzeitig der pH-Wert mit $H_2SO_4$ auf 2.0 eingestellt. Die Biomasse wurde durch Zentrifugation abgetrennt und bei -70 °C gefroren aufbewahrt.

**Beispiel 3: Extraktion**

Säureinaktivierte Biomasse (etwa 0.5 kg) wurde in 500 ml 1%iger Essigsäure mit Hilfe eines Polytron-Homogenisators suspendiert und 1 Stunde bei 0 °C gerührt. Polyethylenimin (50%ige Stammlösung, Serva, Heidelberg) wurde bis zu einer Endkonzentration von 0.25% (w/v) zugegeben. Die Suspension wurde mit 5 N NaOH auf einen pH von 10.0 eingestellt und weitere 2 Stunden bei 0 °C gerührt. Nach Einstellung des pH-Wertes auf 7.5 mit 5 N HCl wurden die Bakterien durch Zentrifugation bei 17000 x g (Beckmann J2-21 Zentrifuge) abgetrennt. Die durchschnittliche Extraktionsausbeute betrug 29.3 ± 5.9% des Gesamtgehaltes an IFN$\alpha$2c.

**Beispiel 4: Chromatographische Reinigung**

*Adsorptionschromatographie auf Silicagel*

Der IFN$\alpha$-haltige Überstand nach der Abtrennung des Bakterienpellets in Beispiel 3 wurde auf eine Silicagel-Säule geladen (Grace, Silica Typ 953W; 35 mg Protein/ml Säulenmaterial, Flußgeschwindigkeit 25 ml/min), die mit 20 mM Tris-HCl, pH 7.5, equilibriert worden war. Die Säule wurde mit 30 Säulenvolumina Startpuffer gewaschen, dann folgte ein Waschschritt mit 20 mM Tris-HCl, 100 mM Tetramethylammoniumchlorid (TMAC), pH 7.5. IFN$\alpha$2c konnte durch Steigerung der TMAC-Konzentration auf 800 mM TMAC eluiert werden (Fig. 3A).

*Hydrophobe Interaktionschromatographie*

Das Material, das von der Silicalgelsäule eluiert wurde, wurde durch Zugabe von festem $(NH_4)_2SO_4$ auf eine Ammoniumsulfatkonzentration von 20% (w/v) eingestellt und auf eine Phenylsepharosesäule (Phenyl Toyopearl, 650S, Tosohaas) geladen, die mit 20 mM Tris-HCl, 30% Ammoniumsulfat equilibriert worden war. IFN$\alpha$2c wurde mit einem linearen Gradienten von 100% Ladebedingungen bis 100% 20mM Tris-HCl, 30% Ethylenglykol, pH 7.5, bei einer Flußgeschindigkeit von 15 ml/min eluiert. Die Reinheit des IFN$\alpha$-Pools betrug 71 ± 15%.

*Kationenaustauschchromatographie*

Das Eluat der hydrophoben Interaktionschromatographie wurde durch extensive Dialyse auf 20 mM Na-Succinat, pH 5.0, eingestellt. Der endgültige pH wurde mit HCl auf 3.0 eingestellt, bevor die Probe auf ein Sulfopropyl-Ionenaustauscherharz (Toyopearl TSK SP 5PW, Tosohaas), equilibriert mit 20 mM Na-Succinat, pH 5.0, geladen wurde. IFNα2c wurde mit einem linearen Gradienten von 100% Ladebedingungen bis 100% 20 mM Na-Succinat, 500 mM NaCl, 10% Ethylenglykol, pH 5.5 (Lösungsmittel B) mit einer Flußgeschindigkeit von 6 ml/min von der Säule eluiert. Das von dieser Säule eluierte IFNα2c hatte routinemäßig eine größere Reinheit als 95%.

*Anionenaustauschchromatographie*

Der IFNα-Pool wurde gegen 10 mM bisTris, pH 5.8, dialysiert und auf eine DEAE-Sepharose (DEAE-Sepharose FastFlow, Pharmacia) geladen, die mit dem gleichen Puffer equilibriert war. Die Elution von IFNα2c erfolgte mit einem linearen Gradienten auf 10 mM bisTris, 500 mM NaCl, 0.1% Tween 20, pH 5.8 (Lösungsmittel B), Fließgeschindigkeit 5 ml/min.

**Beispiel 5: Analyse der IFNα2c-Präparationen**

*Reversed-Phase-HPLC*

Intaktes IFNα2c wurde mit einer BakerBond -WP- C18-Säule [250 x 4.5 mm, Partikelgröße 5 µm] bei 30°C analysiert. Für die Trennung von tryptischen Peptiden wurde eine Merck Supersphere 120-4 C-18-Säule [125 x 4.5 mm, Partikelgröße 4 µm] bei 37°C verwendet. Die Proben wurden unter Verwendung der Lösungsmittel A, 0.1% Trifluoressigsäure in Wasser, und B, 0.1% Trifluoressigsäure in Acetonitril und mit den Gradienten wie in der jeweiligen Abbildungslegende beschrieben chromatographiert.

*SDS-Polyacrylamid-Gelelektrophorese*

IFNα2c-Proben wurden auf 16%-SDS-Polyacrylamid-Gelen unter Standardbedingungen analysiert. Proben wurden vor der Elektrophorese mit Dithiotreitol reduziert. Proteinbanden wurden mit Coomassie-Blue-Färbung visualisiert.

*Quantifizierung von IFNα2c durch ELISA*

Der IFNα2c-Gehalt verschiedener Proben, die während der Reinigung anfielen, wurde mit einem Sandwich-ELISA mit den monoklonalen Antikörpern OMG-2 und MG-7 (Adolf *et al.*, 1990) bestimmt.

**Literatur**

Adolf, G.R., Virology **175**, 410-417 (1990)
Bodo, G. & Maurer-Fogy, I., in: The Biology of the Interferon System 1985 (Hrsg.: Stewart. W.E., II & Schellekens, H.), S. 59-64, Elsevier Scientific Publishing Co., Amsterdam (1985)
Breitling R., Gerlach D., Hartmann M., Behnke D., Mol. Gen. Genet. **217**, 384-391 (1989)
Chang C.N., Kuang W.-J. and E.Y. Chen, Gene **44**, 121-125 (1986)
Dworkin-Rastl E., Swetly P., Dworkin M.B., Gene **21**, 237-248 (1983)
Fuh G., Mulkerrin M.G., Bass S., McFarland M., Brochier M., Bourell J.H., Light D.R., Wells J.A., J. Biol. Chem. **265**, 3111-3115 (1990)
Goeddel D.V., Yelverton E., Ullrich A., Heynecker H.L., Miozarri G., Holmes W., Seeburg P.H., Dull T., May L., Stebbing N., Crea R., Maeda S., McCandliss R., Sloma A., Tabor J.M., Gross M., Familletti P.C., Pestka S., Nature **287**, 411-416 (1980)
Goeddel D.V., Leung D.W., Dull T.J., Gross M., Lawn R.M., McCandliss R., Seeburg P.H., Ullrich A., Yelverton E., Gray P., Nature **290**, 20-26 (1981)
Hauptmann R. and P. Swetly, Nucl. Acids Res. **13**, 4739-4749 (1985)
Ho S.N., Hunt S.N., Horton R.M., Pullen J.K. and L.R. Pease, Gene **77**, 51-59 (1989)
Lee C.H., Mosely S.L., Moon H.W., Whipp S.C., Gyles C.L. and M. So, Infection and Immunity **42**, 264-268 (1983)
Mantei N., Schwarzstein M., Streuli M., Panem S., Nagata S., Weissmann C., Gene **10**, 1-10 (1980)
Picken R.N., Mazaitis A.J., Maas W.K., Rey M. and H. Heyneker, Infection and Immunity **42**, 269-275 (1983)
Sambrook J., Fritsch E.F., Maniatis T., "Molecular cloning - a laboratory manual", Cold Spring Harbor Laboratory Press, Cold Spring Harbor (1989)
Shuttleworth M., Taylor J. and N. Minton, Nucl. Acids Res. **14**, 8689 (1986)

Streuli M., Nagata S., Weissmann C., Science **209**, 1343-1347 (1980)
Thatcher D.R., Panayotatos N., Methods Enzymol. **119**, 166-177 (1986)
Twigg A.J. and D. Sherratt, Nature **283**, 216-218 (1980)

## Patentansprüche

1. Verfahren zur Herstellung von Interferon-α durch Expression in *E. coli*, dadurch gekennzeichnet, daß
   a) Interferon-α exprimiert wird in Zellen, die einen Vektor enthalten, in dem die Signalsequenz des Gens für das hitzestabile Enterotoxin II (STII) aus *E. coli* verknüpft ist mit einer Sequenz, die für reifes menschliches Interferon-α kodiert
   b) das exprimierte Interferon-α isoliert wird.

2. Verfahren nach Anspruch 1, dadurch gekennzeichnet, daß der Vektor zusätzlich einen Promotor für die alkalische Phosphatase (*pho*A) aus *E. coli* enthält.

3. Verfahren nach den Ansprüchen 1 bis 2, dadurch gekennzeichnet, daß der Vektor zusätzlich die Sequenz für die Ribosomenbindungsstelle des STII-Gens enthält.

4. Verfahren nach Anspruch 1 bis 3, dadurch gekennzeichnet, daß die Isolierung des Interferons die Schritte
   a) Adsorptionschromatographie auf Silicagel
   b) Hydrophobe Interaktions-Chromatographie
   c) Kationenaustauschchromatographie
   d) Anionenaustauschchromatographie enthält.

5. Verfahren nach Anspruch 4, dadurch gekennzeichnet, daß die hydrophobe Interaktionschromatographie auf einer Phenylsepharose durchgeführt wird.

6. Verfahren nach Anspruch 4, dadurch gekennzeichnet, daß die Kationenaustauschchromatographie auf einem Sulfopropyl-Ionenaustauscher durchgeführt wird.

7. Verfahren nach Anspruch 4, dadurch gekennzeichnet, daß die Anionenaustauschchromatographie auf einer DEAE-Sepharose durchgeführt wird.

8. Verfahren nach den Ansprüchen 1 bis 7, dadurch gekennzeichnet, daß das Interferon-α Interferon-α2 ist.

9. Verfahren nach Anspruch 8, dadurch gekennzeichnet, daß das Interferon-α2 die Aminosäuresequenz

```
Cys Asp Leu Pro Gln Thr His Ser Leu Gly Ser Arg Arg Thr
Leu Met Leu Leu Ala Gln Met Arg Arg Ile Ser Leu Phe Ser
Cys Leu Lys Asp Arg Arg Asp Phe Gly Phe Pro Gln Glu Glu
Phe Gly Asn Gln Phe Gln Lys Ala Glu Thr Ile Pro Val Leu
His Glu Met Ile Gln Gln Ile Phe Asn Leu Phe Ser Thr Lys
Asp Ser Ser Ala Ala Trp Asp Glu Thr Leu Leu Asp Lys Phe
Tyr Thr Glu Leu Tyr Gln Gln Leu Asn Asp Leu Glu Ala Cys
Val Ile Gln Gly Val Gly Val Thr Glu Thr Pro Leu Met Lys
Glu Asp Ser Ile Leu Ala Val Arg Lys Tyr Phe Gln Arg Ile
Thr Leu Tyr Leu Lys Glu Lys Lys Tyr Ser Pro Cys Ala Trp
Glu Val Val Arg Ala Glu Ile Met Arg Ser Phe Ser Leu Ser
Thr Asn Leu Gln Glu Ser Leu Arg Ser Lys Glu
```

   enthält.

10. Verfahren zur Reinigung von Interferon-α, dadurch gekennzeichnet, daß es die Schritte

a) Adsorptionschromatographie auf Silicagel
b) Hydrophobe Interaktions-Chromatographie
c) Kationenaustauschchromatographie
d) Anionenaustauschchromatographie

enthält.

**11.** Verfahren nach Anspruch 10, dadurch gekennzeichnet, daß die hydrophobe Interaktionschromatographie auf einer Phenylsepharose durchgeführt wird.

**12.** Verfahren nach Anspruch 10, dadurch gekennzeichnet, daß die Kationenaustauschchromatographie auf einem Sulfopropyl-Ionenaustauscher durchgeführt wird.

**13.** Verfahren nach Anspruch 10, dadurch gekennzeichnet, daß die Anionenaustauschchromatographie auf einer DEAE-Sepharose durchgeführt wird.

**14.** Verfahren nach Ansprüchen 10 bis 13, dadurch gekennzeichnet, daß das Interferon-α bakteriell exprimiert wurde.

**15.** Verfahren nach den Ansprüchen 10 bis 14, dadurch gekennzeichnet, daß das Interferon-α Interferon-α2 ist.

**16.** Verfahren nach Anspruch 15, dadurch gekennzeichnet, daß das Interferon-α2 die Aminosäuresequenz

```
Cys Asp Leu Pro Gln Thr His Ser Leu Gly Ser Arg Arg Thr
Leu Met Leu Leu Ala Gln Met Arg Arg Ile Ser Leu Phe Ser
Cys Leu Lys Asp Arg Arg Asp Phe Gly Phe Pro Gln Glu Glu
Phe Gly Asn Gln Phe Gln Lys Ala Glu Thr Ile Pro Val Leu
His Glu Met Ile Gln Gln Ile Phe Asn Leu Phe Ser Thr Lys
Asp Ser Ser Ala Ala Trp Asp Glu Thr Leu Leu Asp Lys Phe
Tyr Thr Glu Leu Tyr Gln Gln Leu Asn Asp Leu Glu Ala Cys
Val Ile Gln Gly Val Gly Val Thr Glu Thr Pro Leu Met Lys
Glu Asp Ser Ile Leu Ala Val Arg Lys Tyr Phe Gln Arg Ile
Thr Leu Tyr Leu Lys Glu Lys Lys Tyr Ser Pro Cys Ala Trp
Glu Val Val Arg Ala Glu Ile Met Arg Ser Phe Ser Leu Ser
Thr Asn Leu Gln Glu Ser Leu Arg Ser Lys Glu
```

enthält.

**17.** Vektor zur Expression von Interferon-α in *E. coli*, dadurch gekennzeichnet, daß er die Signalsequenz des STII-Gens in Verknüpfung mit einer Sequenz enthält, die für reifes menschliches Interferon-α kodiert.

**18.** Vektor nach Anspruch 17, dadurch gekennzeichnet, daß er zusätzlich einen *pho*A-Promotor enthält.

**19.** Vektor nach den Ansprüchen 17 bis 18, dadurch gekennzeichnet, daß er zusätzlich die Ribosomenbindungsstelle des STII-Gens enthält.

**20.** Vektor nach den Ansprüchen 17 bis 19, dadurch gekennzeichnet, daß das Interferon-α Interferon-α2 ist.

**21.** Vektor nach den Ansprüchen 17 bis 19, dadurch gekennzeichnet, daß er die Nukleotidsequenz

TGT GAT CTG CCT CAA ACC CAC AGC CTG GGT AGC AGG AGG ACC
TTG ATG CTC CTG GCA CAG ATG AGG AGA ATC TCT CTT TTC TCC
TGC TTG AAG GAC AGA CGT GAC TTT GGA TTT CCC CAG GAG GAG
TTT GGC AAC CAG TTC CAA AAG GCT GAA ACC ATC CCT GTC CTC
CAT GAG ATG ATC CAG CAG ATC TTC AAT CTC TTC AGC ACA AAG
GAC TCA TCT GCT GCT TGG GAT GAG ACC CTC CTA GAC AAA TTC
TAC ACT GAA CTC TAC CAG CAG CTG AAT GAC CTG GAA GCC TGT
GTG ATA CAG GGG GTG GGG GTG ACA GAG ACT CCC CTG ATG AAG
GAG GAC TCC ATT CTG GCT GTG AGG AAA TAC TTC CAA AGA ATC
ACT CTC TAT CTG AAA GAG AAG AAA TAC AGC CCT TGT GCC TGG
GAG GTT GTC AGA GCA GAA ATC ATG AGA TCT TTT TCT TTG TCA
ACA AAC TTG CAA GAA AGT TTA AGA AGT AAG GAA

oder eine Sequenz, die zu dieser Sequenz zu mehr als 70 % homolog ist und für Interferon-α kodiert, enthält.

**22.** Vektor nach Anspruch 21, dadurch gekennzeichnet, daß er die Nukleotidsequenz

GAATTCGAGATTATCGTCACTGCAATGCTTCGCAATATGGCGCAAAATGACCAACAG
CGGTTGATTGATCAGGTAGAGGGGGCGCTGTACGAGGTAAAGCCCGATGCCAGCATT
CCTGACGACGATACGGAGCTGCTGCGCGATTACGTAAAGAAGTTATTGAAGCATCCT
CGTCAGTAAAAAGTTAATCTTTTCAACAGCTGTCATAAAGTTGTCACGGCCGAGACT
TATAGTCGCTTTGTTTTTATTTTTTAATGTATTTGCTCGAGAGGTTGAGGTGATTTT
ATG AAA AAG AAT ATC GCA TTT CTT CTT GCA TCT ATG TTC GTT
TTT TCT ATT GCT ACA AAT GCC TAT GCA TGT GAT CTG CCT CAA
ACC CAC AGC CTG GGT AGC AGG AGG ACC TTG ATG CTC CTG GCA
CAG ATG AGG AGA ATC TCT CTT TTC TCC TGC TTG AAG GAC AGA
CGT GAC TTT GGA TTT CCC CAG GAG GAG TTT GGC AAC CAG TTC
CAA AAG GCT GAA ACC ATC CCT GTC CTC CAT GAG ATG ATC CAG
CAG ATC TTC AAT CTC TTC AGC ACA AAG GAC TCA TCT GCT GCT
TGG GAT GAG ACC CTC CTA GAC AAA TTC TAC ACT GAA CTC TAC
CAG CAG CTG AAT GAC CTG GAA GCC TGT GTG ATA CAG GGG GTG

GGG GTG ACA GAG ACT CCC CTG ATG AAG GAG GAC TCC ATT CTG
GCT GTG AGG AAA TAC TTC CAA AGA ATC ACT CTC TAT CTG AAA
GAG AAG AAA TAC AGC CCT TGT GCC TGG GAG GTT GTC AGA GCA
GAA ATC ATG AGA TCT TTT TCT TTG TCA ACA AAC TTG CAA GAA
AGT TTA AGA AGT AAG GAA TGATAACGATCGTAACTGCA

enthält.

**23.** Verwendung des Vektors gemäß einem der Ansprüche 17 bis 22 zur Herstellung von Interferon-α.

A)

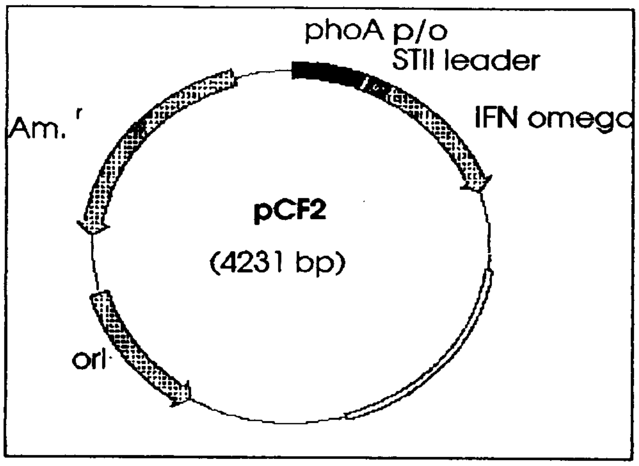


B)

```
gaattggagattatcgtcactgcaatgcttcgcaatatggcgcaaaatgaccaac     55
agcggttgattgatcaggtagagggggcgctgtacgaggtaaagcccgatgccag     110
cattcctgacgacgatacggagctgctgcgcgattacgtaaagaagttattgaag     165
catcctcgtcagtaaaaagttaatcttttcaacagctgtcataaagttgtcacgg     220
ccgagacttatagtcgctttgtttttatttttaatgtatttgctcgagaggttg      275
aggtgatttt  ATG AAA AAG AAT ATC GCA TTT CTT CTT GCA TCT     318
             M   K   K   N   I   A   F   L   L   A   S      11
ATG TTC GTT TTT TCT ATT GCT ACA AAT GCC TAT GCA TGT GAT     360
 M   F   V   F   S   I   A   T   N   A   Y   A   C   D      25
CTG CCT CAG AAC CAT GGC CTA CTT AGC AGG AAC ACC TTG GTG     402
 L   P   Q   N   H   G   L   L   S   R   N   T   L   V      39
CTT CTG CAC CAA ATG AGG AGA ATC TCC CCT TTC TTG TGT CTC     444
 L   L   H   Q   M   R   R   I   S   P   F   L   C   L      53
AAG GAC AGA AGA GAC TTC AGG TTC CCC CAG GAG ATG GTA AAA     486
 K   D   R   R   D   F   R   F   P   Q   E   M   V   K      67
GGG AGC CAG TTG CAG AAG GCC CAT GTC ATG TCT GTC CTC CAT     528
 G   S   Q   L   Q   K   A   H   V   M   S   V   L   H      81
GAG ATG CTG CAG CAG ATC TTC AGC CTC TTC CAC ACA GAG CGC     570
 E   M   L   Q   Q   I   F   S   L   F   H   T   E   R      95
TCC TCT GCT GCC TGG AAC ATG ACC CTC CTA GAC CAA CTC CAC     612
 S   S   A   A   W   N   M   T   L   L   D   Q   L   H      109
ACT GGA CTT CAT CAG CAA CTG CAA CAC CTG GAG ACC TGC TTG     654
 T   G   L   H   Q   Q   L   Q   H   L   E   T   C   L      123
CTG CAG GTA GTG GGA GAA GGA GAA TCT GCT GGG GCA ATT AGC     696
 L   Q   V   V   G   E   G   E   S   A   G   A   I   S      137
AGC CCT GCA CTG ACC TTG AGG AGG TAC TTC CAG GGA ATC CGT     738
 S   P   A   L   T   L   R   R   Y   F   Q   G   I   R      151
GTC TAC CTG AAA GAG AAG AAA TAC AGC GAC TGT GCC TGG GAA     780
 V   Y   L   K   E   K   K   Y   S   D   C   A   W   E      165
GTT GTC AGA ATG GAA ATC ATG AAA TCC TTG TTC TTA TCA ACA     822
 V   V   R   M   E   I   M   K   S   L   F   L   S   T      179
AAC ATG CAA GAA AGA CTG AGA AGT AAA GAT AGA GAC CTG GGC     864
 N   M   Q   E   R   L   R   S   K   D   R   D   L   G      193
TCA TCT TGA aatgattctcattgattaatttgccatataacacttgcacatg     916
 S   S   *                                                  196
tgactctggtcaattcaaaagactcttatttcggctttaatcacagaattgactg     971
aattagttctgcaaatactttgtcggtatattaagccagtatatgttaaaaagac     1026
ttaggttcaggggcatcagtccctaagatgttatttatttttactcatttattta     1081
ttcttacattttatcatatttatactatttatattcttatataacaaatgtttgc     1136
ctttacattgtattaagataacaaaacatgttcaggatcc                    1176
```

**Fig. 1**

A)

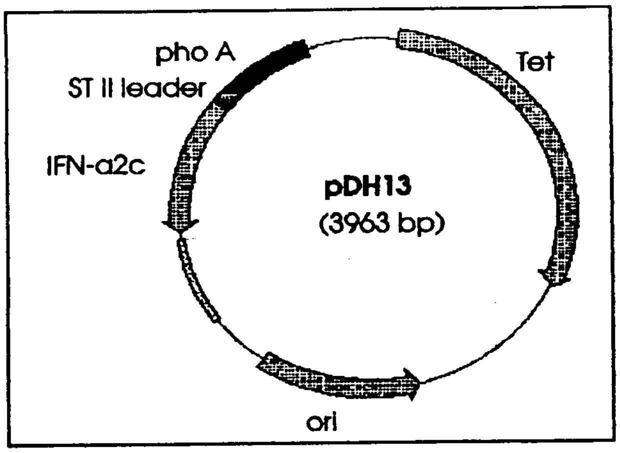


B)

EcoRI

gaattcgagattatcgtcactgcaatgcttcgcaatatggcgcaaaatgaccaac 55
agcggttgattgatcaggtagaggggcgctgtacgaggtaaagcccgatgccag 110
cattcctgacgacgatacggagctgctgcgcgattacgtaaagaagttattgaag 165
catcctcgtcagtaaaaagttaatcttttcaacagctgtcataaagttgtcacgg 220

XhoI

ccgagacttatagtcgctttgtttttatttttaatgtatttgctcgagaggttg 275

STII Leader peptide ->

aggtgatttt ATG AAA AAG AAT ATC GCA TTT CTT CTT GCA TCT 318
M K K N I A F L L A S 11

IFNα2c ->

ATG TTC GTT TTT TCT ATT GCT ACA AAT GCC TAT GCA TGT GAT 360
M F V F S I A T N A Y A C D 25
CTG CCT CAA ACC CAC AGC CTG GGT AGC AGG AGG ACC TTG ATG 402
L P Q T H S L G S R R T L M 39
CTC CTG GCA CAG ATG AGG AGA ATC TCT CTT TTC TCC TGC TTG 444
L L A Q M R R I S L F S C L 53
AAG GAC AGA CGT GAC TTT GGA TTT CCC CAG GAG GAG TTT GGC 486
K D R R D F G F P Q E E F G 67
AAC CAG TTC CAA AAG GCT GAA ACC ATC CCT GTC CTC CAT GAG 528
N Q F Q K A E T I P V L H E 81
ATG ATC CAG CAG ATC TTC AAT CTC TTC AGC ACA AAG GAC TCA 570
M I Q Q I F N L F S T K D S 95
TCT GCT GCT TGG GAT GAG ACC CTC CTA GAC AAA TTC TAC ACT 612
S A A W D E T L L D K F Y T 109
GAA CTC TAC CAG CAG CTG AAT GAC CTG GAA GCC TGT GTG ATA 654
E L Y Q Q L N D L E A C V I 123
CAG GGG GTG GGG GTG ACA GAG ACT CCC CTG ATG AAG GAG GAC 696
Q G V G V T E T P L M K E D 137
TCC ATT CTG GCT GTG AGG AAA TAC TTC CAA AGA ATC ACT CTC 738
S I L A V R K Y F Q R I T L 151
TAT CTG AAA GAG AAG AAA TAC AGC CCT TGT GCC TGG GAG GTT 780
Y L K E K K Y S P C A W E V 165
GTC AGA GCA GAA ATC ATG AGA TCT TTT TCT TTG TCA ACA AAC 822
V R A E I M R S F S L S T N 179

PvuI PstI

TTG CAA GAA AGT TTA AGA AGT AAG GAA tgataacgatcgtaactgc 868
L Q E S L R S K E 188

HindIII

agaagctt 876

**Fig. 2**

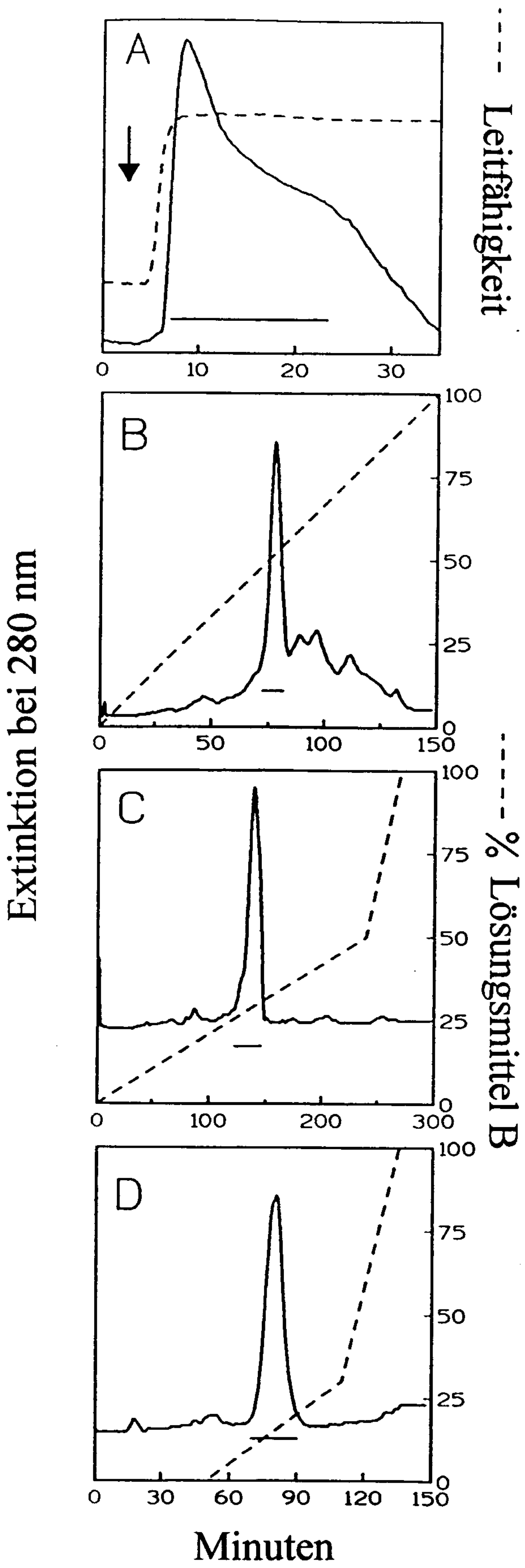
A
B
C
D
Leitfähigkeit
Extinktion bei 280 nm
% Lösungsmittel B
0
10
20
30
50
100
150
200
300
60
90
120
25
75
Minuten

**Fig. 3**

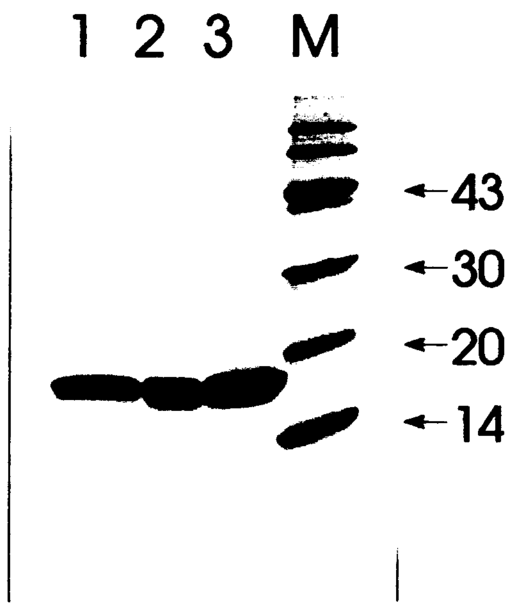


Fig. 4

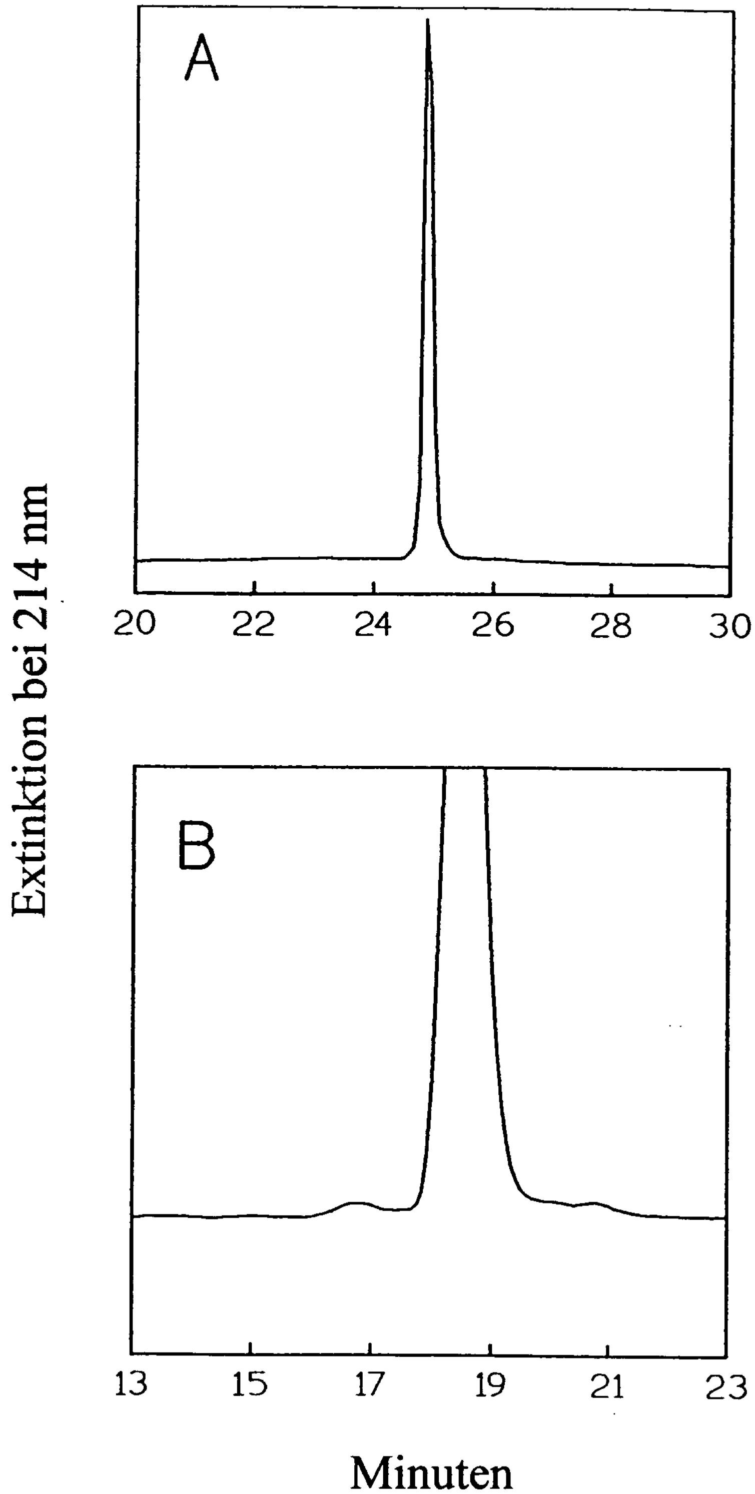
A
B
Extinktion bei 214 nm
20
22
24
26
28
30
13
15
17
19
21
23
Minuten

**Fig. 5**

SEQUENZPROTOKOLL

(1) ALGEMEINE INFORMATION:

(i) ANMELDER:
(A) NAME: Boehringer Ingelheim International GmbH
(B) STRASSE: Binger Strasse
(C) ORT: Ingelheim
(E) LAND: Germany
(F) POSTLEITZAHL: 55218
(G) TELEPHON: 49-6132-77-2770
(H) TELEFAX: 49-6132-77-4377

(ii) ANMELDETITEL: Verfahren zur Herstellung und Reinigung von Interferon-alpha

(iii) ANZAHL DER SEQUENZEN: 12

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 25 Basenpaare
(B) ART: Nukleinsäure
(C) STRANGFORM: beides
(D) TOPOLOGIE: nicht bekannt

(ii) ART DES MOLEKÜLS: cDNS

(xi) SEQUENZBESCHREIBUNG: SEQ ID NO: 1:

CGTCTTCAAG AATTCGAGAT TATCG 25

(2) INFORMATION ZU SEQ ID NO: 2:

(i) SEQUENZ CHARAKTERISTIKA:
(A) LÄNGE: 56 Basenpaare
(B) ART: Nukleinsäure
(C) STRANGFORM: beides
(D) TOPOLOGIE: nicht bekannt

(ii) ART DES MOLEKÜLS: cDNS

(xi) SEQUENZBESCHREIBUNG: SEQ ID NO: 2:

GACTTCAGAA GCTTCTGCAG TTACGATCGT TATCATTCCT TACTTCTTAA ACTTTC 56

(2) INFORMATION ZU SEQ ID NO: 3:

(i) SEQUENZ CHARAKTERISTIKA:
(A) LÄNGE: 35 Basenpaare
(B) ART: Nukleinsäure
(C) STRANGFORM: beides

(D) TOPOLOGIE: nicht bekannt

(ii) ART DES MOLEKÜLS: cDNS

(xi) SEQUENZBESCHREIBUNG: SEQ ID NO: 3:

ATGCCTATGC ATGTGATCTG CCTCAAACCC ACAGC 35

(2) INFORMATION ZU SEQ ID NO: 4:

(i) SEQUENZ CHARAKTERISTIKA:
(A) LÄNGE: 34 Basenpaare
(B) ART: Nukleinsäure
(C) STRANGFORM: beides
(D) TOPOLOGIE: nicht bekannt

(ii) ART DES MOLEKÜLS: cDNS

(xi) SEQUENZBESCHREIBUNG: SEQ ID NO: 4:

GGCAGATCAC ATGCATAGGC ATTTGTAGCA ATAG 34

(2) INFORMATION ZU SEQ ID NO: 5:

(i) SEQUENZ CHARAKTERISTIKA:
(A) LÄNGE: 165 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 5:

Cys Asp Leu Pro Gln Thr His Ser Leu Gly Ser Arg Arg Thr Leu Met
1 5 10 15

Leu Leu Ala Gln Met Arg Arg Ile Ser Leu Phe Ser Cys Leu Lys Asp
20 25 30

Arg Arg Asp Phe Gly Phe Pro Gln Glu Glu Phe Gly Asn Gln Phe Gln
35 40 45

Lys Ala Glu Thr Ile Pro Val Leu His Glu Met Ile Gln Gln Ile Phe
50 55 60

Asn Leu Phe Ser Thr Lys Asp Ser Ser Ala Ala Trp Asp Glu Thr Leu
65 70 75 80

Leu Asp Lys Phe Tyr Thr Glu Leu Tyr Gln Gln Leu Asn Asp Leu Glu
85 90 95

Ala Cys Val Ile Gln Gly Val Gly Val Thr Glu Thr Pro Leu Met Lys
100 105 110

Glu Asp Ser Ile Leu Ala Val Arg Lys Tyr Phe Gln Arg Ile Thr Leu
115 120 125

Tyr Leu Lys Glu Lys Lys Tyr Ser Pro Cys Ala Trp Glu Val Val Arg

```
        130                    135                    14C

    Ala Glu Ile Met Arg Ser Phe Ser Leu Ser Thr Asn Leu Gln Glu Ser
    145                 150                 155                 160

    Leu Arg Ser Lys Glu
                    165
```

(2) INFORMATION ZU SEQ ID NO: 6:

(i) SEQUENZ CHARAKTERISTIKA:
(A) LÄNGE: 495 Basenpaare
(B) ART: Nukleinsäure
(C) STRANGFORM: beides
(D) TOPOLOGIE: nicht bekannt

(ii) ART DES MOLEKÜLS: cDNS

(ix) MERKMALE:
(A) NAME/SCHLÜSSEL: CDS
(B) LAGE: 1..495

(xi) SEQUENZBESCHREIBUNG: SEQ ID NO: 6:

```
TGT GAT CTG CCT CAA ACC CAC AGC CTG GGT AGC AGG AGG ACC TTG ATG        48
Cys Asp Leu Pro Gln Thr His Ser Leu Gly Ser Arg Arg Thr Leu Met
  1               5                  10                  15

CTC CTG GCA CAG ATG AGG AGA ATC TCT CTT TTC TCC TGC TTG AAG GAC        96
Leu Leu Ala Gln Met Arg Arg Ile Ser Leu Phe Ser Cys Leu Lys Asp
            20                  25                  30

AGA CGT GAC TTT GGA TTT CCC CAG GAG GAG TTT GGC AAC CAG TTC CAA       144
Arg Arg Asp Phe Gly Phe Pro Gln Glu Glu Phe Gly Asn Gln Phe Gln
        35                  40                  45

AAG GCT GAA ACC ATC CCT GTC CTC CAT GAG ATG ATC CAG CAG ATC TTC       192
Lys Ala Glu Thr Ile Pro Val Leu His Glu Met Ile Gln Gln Ile Phe
    50                  55                  60

AAT CTC TTC AGC ACA AAG GAC TCA TCT GCT GCT TGG GAT GAG ACC CTC       240
Asn Leu Phe Ser Thr Lys Asp Ser Ser Ala Ala Trp Asp Glu Thr Leu
 65                  70                  75                  80

CTA GAC AAA TTC TAC ACT GAA CTC TAC CAG CAG CTG AAT GAC CTG GAA       288
Leu Asp Lys Phe Tyr Thr Glu Leu Tyr Gln Gln Leu Asn Asp Leu Glu
                85                  90                  95

GCC TGT GTG ATA CAG GGG GTG GGG GTG ACA GAG ACT CCC CTG ATG AAG       336
Ala Cys Val Ile Gln Gly Val Gly Val Thr Glu Thr Pro Leu Met Lys
            100                 105                 110

GAG GAC TCC ATT CTG GCT GTG AGG AAA TAC TTC CAA AGA ATC ACT CTC       384
Glu Asp Ser Ile Leu Ala Val Arg Lys Tyr Phe Gln Arg Ile Thr Leu
        115                 120                 125

TAT CTG AAA GAG AAG AAA TAC AGC CCT TGT GCC TGG GAG GTT GTC AGA       432
Tyr Leu Lys Glu Lys Lys Tyr Ser Pro Cys Ala Trp Glu Val Val Arg
    130                 135                 140

GCA GAA ATC ATG AGA TCT TTT TCT TTG TCA ACA AAC TTG CAA GAA AGT       480
Ala Glu Ile Met Arg Ser Phe Ser Leu Ser Thr Asn Leu Gln Glu Ser
```

```
145                 150                 155                 160

TTA AGA AGT AAG GAA                                                   495
Leu Arg Ser Lys Glu
                165
```

(2) INFORMATION ZU SEQ ID NO: 7:

(i) SEQUENZ CHARAKTERISTIKA:
(A) LÄNGE: 165 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 7:

```
Cys Asp Leu Pro Gln Thr His Ser Leu Gly Ser Arg Arg Thr Leu Met
  1               5                  10                  15

Leu Leu Ala Gln Met Arg Arg Ile Ser Leu Phe Ser Cys Leu Lys Asp
            20                  25                  30

Arg Arg Asp Phe Gly Phe Pro Gln Glu Glu Phe Gly Asn Gln Phe Gln
        35                  40                  45

Lys Ala Glu Thr Ile Pro Val Leu His Glu Met Ile Gln Gln Ile Phe
    50                  55                  60

Asn Leu Phe Ser Thr Lys Asp Ser Ser Ala Ala Trp Asp Glu Thr Leu
65                  70                  75                  80

Leu Asp Lys Phe Tyr Thr Glu Leu Tyr Gln Gln Leu Asn Asp Leu Glu
                85                  90                  95

Ala Cys Val Ile Gln Gly Val Gly Val Thr Glu Thr Pro Leu Met Lys
            100                 105                 110

Glu Asp Ser Ile Leu Ala Val Arg Lys Tyr Phe Gln Arg Ile Thr Leu
        115                 120                 125

Tyr Leu Lys Glu Lys Lys Tyr Ser Pro Cys Ala Trp Glu Val Val Arg
    130                 135                 140

Ala Glu Ile Met Arg Ser Phe Ser Leu Ser Thr Asn Leu Gln Glu Ser
145                 150                 155                 160

Leu Arg Ser Lys Glu
                165
```

(2) INFORMATION ZU SEQ ID NO: 8:

(i) SEQUENZ CHARAKTERISTIKA:
(A) LÄNGE: 869 Basenpaare
(B) ART: Nukleinsäure
(C) STRANGFORM: beides
(D) TOPOLOGIE: nicht bekannt

(ii) ART DES MOLEKÜLS: cDNS

(xi) SEQUENZBESCHREIBUNG: SEQ ID NO: 8:

GAATTCGAGA TTATCGTCAC TGCAATGCTT CGCAATATGG CGCAAAATGA CCAACAGCGG 60

TTGATTGATC AGGTAGAGGG GGCGCTGTAC GAGGTAAAGC CCGATGCCAG CATTCCTGAC 120

GACGATACGG AGCTGCTGCG CGATTACGTA AAGAAGTTAT TGAAGCATCC TCGTCAGTAA 180

AAAGTTAATC TTTTCAACAG CTGTCATAAA GTTGTCACGG CCGAGACTTA TAGTCGCTTT 240

GTTTTTATTT TTTAATGTAT TTGCTCGAGA GGTTGAGGTG ATTTTATGAA AAAGAATATC 300

GCATTTCTTC TTGCATCTAT GTTCGTTTTT TCTATTGCTA CAAATGCCTA TGCATGTGAT 360

CTGCCTCAAA CCCACAGCCT GGGTAGCAGG AGGACCTTGA TGCTCCTGGC ACAGATGAGG 420

AGAATCTCTC TTTTCTCCTG CTTGAAGGAC AGACGTGACT TTGGATTTCC CCAGGAGGAG 480

TTTGGCAACC AGTTCCAAAA GGCTGAAACC ATCCCTGTCC TCCATGAGAT GATCCAGCAG 540

ATCTTCAATC TCTTCAGCAC AAAGGACTCA TCTGCTGCTT GGGATGAGAC CCTCCTAGAC 600

AAATTCTACA CTGAACTCTA CCAGCAGCTG AATGACCTGG AAGCCTGTGT GATACAGGGG 660

GTGGGGGTGA CAGAGACTCC CCTGATGAAG GAGGACTCCA TTCTGGCTGT GAGGAAATAC 720

TTCCAAAGAA TCACTCTCTA TCTGAAAGAG AAGAAATACA GCCCTTGTGC CTGGGAGGTT 780

GTCAGAGCAG AAATCATGAG ATCTTTTTCT TTGTCAACAA ACTTGCAAGA AAGTTTAAGA 840

AGTAAGGAAT GATAACGATC GTAACTGCA 869

(2) INFORMATION ZU SEQ ID NO: 9:

(i) SEQUENZ CHARAKTERISTIKA:
(A) LÄNGE: 1177 Basenpaare
(B) ART: Nukleinsäure
(C) STRANGFORM: beides
(D) TOPOLOGIE: nicht bekannt

(ii) ART DES MOLEKÜLS: cDNS

(ix) MERKMALE:
(A) NAME/SCHLÜSSEL: CDS
(B) LAGE: 286..873
(D) SONSTIGE ANGABEN: /function= "Cytokine"
/product= "Interferon-omega1"

(ix) MERKMALE:
(A) NAME/SCHLÜSSEL: mat_peptide
(B) LAGE: 355..873
(D) SONSTIGE ANGABEN: /function= "Cytokine"
/product= "Interferon-omega"

(ix) MERKMALE:
(A) NAME/SCHLÜSSEL: sig_peptide
(B) LAGE: 286..354
(D) SONSTIGE ANGABEN: /product= "ST II Leader"

(xi) SEQUENZBESCHREIBUNG: SEQ ID NO: 9:

GAATTGGAGA TTATCGTCAC TGCAATGCTT CGCAATATGG CGCAAAATGA CCAACAGCGG 60

TTGATTGATC AGGTAGAGGG GGCGCTGTAC GAGGTAAAGC CCGATGCCAG CATTCCTGAC 120

GACGATACGG AGCTGCTGCG CGATTACGTA AAGAAGTTAT TGAAGCATCC TCGTCAGTAA 180

AAAGTTAATC TTTTCAACAG CTGTCATAAA GTTGTCACGG CCGAGACTTA TAGTCGCTTT 240

GTTTTTATTT TTTAATGTAT TTGCTCGAGA GGTTGAGGTG ATTTT ATG AAA AAG 294
Met Lys Lys
-23

AAT ATC GCA TTT CTT CTT GCA TCT ATG TTC GTT TTT TCT ATT GCT ACA 342
Asn Ile Ala Phe Leu Leu Ala Ser Met Phe Val Phe Ser Ile Ala Thr
-20 -15 -10 -5

AAT GCC TAT GCA TGT GAT CTG CCT CAG AAC CAT GGC CTA CTT AGC AGG 390
Asn Ala Tyr Ala Cys Asp Leu Pro Gln Asn His Gly Leu Leu Ser Arg
1 5 10

AAC ACC TTG GTG CTT CTG CAC CAA ATG AGG AGA ATC TCC CCT TTC TTG 438
Asn Thr Leu Val Leu Leu His Gln Met Arg Arg Ile Ser Pro Phe Leu
15 20 25

TGT CTC AAG GAC AGA AGA GAC TTC AGG TTC CCC CAG GAG ATG GTA AAA 486
Cys Leu Lys Asp Arg Arg Asp Phe Arg Phe Pro Gln Glu Met Val Lys
30 35 40

GGG AGC CAG TTG CAG AAG GCC CAT GTC ATG TCT GTC CTC CAT GAG ATG 534
Gly Ser Gln Leu Gln Lys Ala His Val Met Ser Val Leu His Glu Met
45 50 55 60

CTG CAG CAG ATC TTC AGC CTC TTC CAC ACA GAG CGC TCC TCT GCT GCC 582
Leu Gln Gln Ile Phe Ser Leu Phe His Thr Glu Arg Ser Ser Ala Ala
65 70 75

TGG AAC ATG ACC CTC CTA GAC CAA CTC CAC ACT GGA CTT CAT CAG CAA 630
Trp Asn Met Thr Leu Leu Asp Gln Leu His Thr Gly Leu His Gln Gln
80 85 90

CTG CAA CAC CTG GAG ACC TGC TTG CTG CAG GTA GTG GGA GAA GGA GAA 678
Leu Gln His Leu Glu Thr Cys Leu Leu Gln Val Val Gly Glu Gly Glu
95 100 105

TCT GCT GGG GCA ATT AGC AGC CCT GCA CTG ACC TTG AGG AGG TAC TTC 726
Ser Ala Gly Ala Ile Ser Ser Pro Ala Leu Thr Leu Arg Arg Tyr Phe
110 115 120

CAG GGA ATC CGT GTC TAC CTG AAA GAG AAG AAA TAC AGC GAC TGT GCC 774
Gln Gly Ile Arg Val Tyr Leu Lys Glu Lys Lys Tyr Ser Asp Cys Ala
125 130 135 140

TGG GAA GTT GTC AGA ATG GAA ATC ATG AAA TCC TTG TTC TTA TCA ACA 822
Trp Glu Val Val Arg Met Glu Ile Met Lys Ser Leu Phe Leu Ser Thr
145 150 155

AAC ATG CAA GAA AGA CTG AGA AGT AAA GAT AGA GAC CTG GGC TCA TCT 870
Asn Met Gln Glu Arg Leu Arg Ser Lys Asp Arg Asp Leu Gly Ser Ser
160 165 170

TGAAATGATT CTCATTGATT AATTTGCCAT ATAACACTTG CACATGTGAC TCTGGTCAAT 930

TCAAAAGACT CTTATTTCGG CTTTAATCAC AGAATTGACT GAATTAGTTC TGCAAATACT 990

TTGTCGGTAT ATTAAGCCAG TATATGTTAA AAAGACTTAG GTTCAGGGGC ATCAGTCCCT 1050

AAGATGTTAT TTATTTTTAC TCATTTATTT ATTCTTACAT TTTATCATAT TTATACTATT 1110

TATATTCTTA TATAACAAAT GTTTGCCTTT ACATTGTATT AAGATAACAA AACATGTTCA 1170

GGATCCA 1177

(2) INFORMATION ZU SEQ ID NO: 10:

(i) SEQUENZ CHARAKTERISTIKA:
(A) LÄNGE: 195 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 10:

```
Met Lys Lys Asn Ile Ala Phe Leu Leu Ala Ser Met Phe Val Phe Ser
-23         -20                 -15                 -10

Ile Ala Thr Asn Ala Tyr Ala Cys Asp Leu Pro Gln Asn His Gly Leu
         -5                   1               5

Leu Ser Arg Asn Thr Leu Val Leu Leu His Gln Met Arg Arg Ile Ser
 10                  15                  20                  25

Pro Phe Leu Cys Leu Lys Asp Arg Arg Asp Phe Arg Phe Pro Gln Glu
                 30                  35                  40

Met Val Lys Gly Ser Gln Leu Gln Lys Ala His Val Met Ser Val Leu
             45                  50                  55

His Glu Met Leu Gln Gln Ile Phe Ser Leu Phe His Thr Glu Arg Ser
         60                  65                  70

Ser Ala Ala Trp Asn Met Thr Leu Leu Asp Gln Leu His Thr Gly Leu
     75                  80                  85

His Gln Gln Leu Gln His Leu Glu Thr Cys Leu Leu Gln Val Val Gly
 90                  95                 100                 105

Glu Gly Glu Ser Ala Gly Ala Ile Ser Ser Pro Ala Leu Thr Leu Arg
                110                 115                 120

Arg Tyr Phe Gln Gly Ile Arg Val Tyr Leu Lys Glu Lys Lys Tyr Ser
            125                 130                 135

Asp Cys Ala Trp Glu Val Val Arg Met Glu Ile Met Lys Ser Leu Phe
        140                 145                 150

Leu Ser Thr Asn Met Gln Glu Arg Leu Arg Ser Lys Asp Arg Asp Leu
    155                 160                 165

Gly Ser Ser
170
```

(2) INFORMATION ZU SEQ ID NO: 11:

(i) SEQUENZ CHARAKTERISTIKA:
(A) LÄNGE: 879 Basenpaare
(B) ART: Nukleinsäure
(C) STRANGFORM: beides

(D) TOPOLOGIE: nicht bekannt

(ii) ART DES MOLEKÜLS: cDNS

(ix) MERKMALE:
(A) NAME/SCHLÜSSEL: CDS
(B) LAGE: 286..852

(ix) MERKMALE:
(A) NAME/SCHLÜSSEL: mat_peptide
(B) LAGE: 355..852
(D) SONSTIGE ANGABEN: /function= "Cytokine"
/product= "Interferon-alpha-2c"

(ix) MERKMALE:
(A) NAME/SCHLÜSSEL: sig_peptide
(B) LAGE: 286..354
(D) SONSTIGE ANGABEN: /product= "ST II Leader"

(xi) SEQUENZBESCHREIBUNG: SEQ ID NO: 11:

```
GAATTCGAGA TTATCGTCAC TGCAATGCTT CGCAATATGG CGCAAAATGA CCAACAGCGG      60

TTGATTGATC AGGTAGAGGG GGCGCTGTAC GAGGTAAAGC CCGATGCCAG CATTCCTGAC     120

GACGATACGG AGCTGCTGCG CGATTACGTA AAGAAGTTAT TGAAGCATCC TCGTCAGTAA     180

AAAGTTAATC TTTTCAACAG CTGTCATAAA GTTGTCACGG CCGAGACTTA TAGTCGCTTT     240

GTTTTTATTT TTTAATGTAT TTGCTCGAGA GGTTGAGGTG ATTTT ATG AAA AAG        294
                                                  Met Lys Lys
                                                  -23

AAT ATC GCA TTT CTT CTT GCA TCT ATG TTC GTT TTT TCT ATT GCT ACA      342
Asn Ile Ala Phe Leu Leu Ala Ser Met Phe Val Phe Ser Ile Ala Thr
-20                 -15                 -10                  -5

AAT GCC TAT GCA TGT GAT CTG CCT CAA ACC CAC AGC CTG GGT AGC AGG      390
Asn Ala Tyr Ala Cys Asp Leu Pro Gln Thr His Ser Leu Gly Ser Arg
                  1               5                  10

AGG ACC TTG ATG CTC CTG GCA CAG ATG AGG AGA ATC TCT CTT TTC TCC      438
Arg Thr Leu Met Leu Leu Ala Gln Met Arg Arg Ile Ser Leu Phe Ser
         15                  20                  25

TGC TTG AAG GAC AGA CGT GAC TTT GGA TTT CCC CAG GAG GAG TTT GGC      486
Cys Leu Lys Asp Arg Arg Asp Phe Gly Phe Pro Gln Glu Glu Phe Gly
     30                  35                  40

AAC CAG TTC CAA AAG GCT GAA ACC ATC CCT GTC CTC CAT GAG ATG ATC      534
Asn Gln Phe Gln Lys Ala Glu Thr Ile Pro Val Leu His Glu Met Ile
 45                  50                  55                  60

CAG CAG ATC TTC AAT CTC TTC AGC ACA AAG GAC TCA TCT GCT GCT TGG      582
Gln Gln Ile Phe Asn Leu Phe Ser Thr Lys Asp Ser Ser Ala Ala Trp
                 65                  70                  75

GAT GAG ACC CTC CTA GAC AAA TTC TAC ACT GAA CTC TAC CAG CAG CTG      630
Asp Glu Thr Leu Leu Asp Lys Phe Tyr Thr Glu Leu Tyr Gln Gln Leu
             80                  85                  90

AAT GAC CTG GAA GCC TGT GTG ATA CAG GGG GTG GGG GTG ACA GAG ACT      678
```

```
Asn Asp Leu Glu Ala Cys Val Ile Gln Gly Val Gly Val Thr Glu Thr
         95                     1CC                 105

CCC CTG ATG AAG GAG GAC TCC ATT CTG GCT GTG AGG AAA TAC TTC CAA        726
Pro Leu Met Lys Glu Asp Ser Ile Leu Ala Val Arg Lys Tyr Phe Gln
    110                 115                 120

AGA ATC ACT CTC TAT CTG AAA GAG AAG AAA TAC AGC CCT TGT GCC TGG        774
Arg Ile Thr Leu Tyr Leu Lys Glu Lys Lys Tyr Ser Pro Cys Ala Trp
125                 130                 135                 140

GAG GTT GTC AGA GCA GAA ATC ATG AGA TCT TTT TCT TTG TCA ACA AAC        822
Glu Val Val Arg Ala Glu Ile Met Arg Ser Phe Ser Leu Ser Thr Asn
                145                 150                 155

TTG CAA GAA AGT TTA AGA AGT AAG GAA TGATAACGAT CGTAACTGCA              869
Leu Gln Glu Ser Leu Arg Ser Lys Glu
            160                 165

GAAGCTTAAT                                                             879
```

(2) INFORMATION ZU SEQ ID NO: 12:

(i) SEQUENZ CHARAKTERISTIKA:
(A) LÄNGE: 188 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 12:

```
Met Lys Lys Asn Ile Ala Phe Leu Leu Ala Ser Met Phe Val Phe Ser
-23         -20                 -15                 -10

Ile Ala Thr Asn Ala Tyr Ala Cys Asp Leu Pro Gln Thr His Ser Leu
        -5                  1                   5

Gly Ser Arg Arg Thr Leu Met Leu Leu Ala Gln Met Arg Arg Ile Ser
 10                 15                  20                  25

Leu Phe Ser Cys Leu Lys Asp Arg Arg Asp Phe Gly Phe Pro Gln Glu
                30                  35                  40

Glu Phe Gly Asn Gln Phe Gln Lys Ala Glu Thr Ile Pro Val Leu His
            45                  50                  55

Glu Met Ile Gln Gln Ile Phe Asn Leu Phe Ser Thr Lys Asp Ser Ser
        60                  65                  70

Ala Ala Trp Asp Glu Thr Leu Leu Asp Lys Phe Tyr Thr Glu Leu Tyr
    75                  80                  85

Gln Gln Leu Asn Asp Leu Glu Ala Cys Val Ile Gln Gly Val Gly Val
 90                 95                  100                 105

Thr Glu Thr Pro Leu Met Lys Glu Asp Ser Ile Leu Ala Val Arg Lys
                110                 115                 120

Tyr Phe Gln Arg Ile Thr Leu Tyr Leu Lys Glu Lys Lys Tyr Ser Pro
            125                 130                 135

Cys Ala Trp Glu Val Val Arg Ala Glu Ile Met Arg Ser Phe Ser Leu

                140                 145                 15C

Ser Thr Asn Leu Gln Glu Ser Leu Arg Ser Lys Glu
    155                 160                 165
```